(19) **Europäisches Patentamt / European Patent Office / Office européen des brevets**

(11) **EP 1 985 582 A1**

(12) # EUROPEAN PATENT APPLICATION
published in accordance with Art. 158(3) EPC

(43) Date of publication:
**29.10.2008 Bulletin 2008/44**

(21) Application number: **07708382.2**

(22) Date of filing: **14.02.2007**

(51) Int Cl.:
*C01B 11/02* (2006.01)   *A01N 25/12* (2006.01)
*A01N 59/00* (2006.01)   *A01N 59/08* (2006.01)
*A01P 3/00* (2006.01)   *A61L 9/01* (2006.01)
*B01J 7/00* (2006.01)

(86) International application number:
**PCT/JP2007/052580**

(87) International publication number:
**WO 2007/094345 (23.08.2007 Gazette 2007/34)**

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **17.02.2006   JP 2006040955**

(71) Applicant: **Taiko Pharmaceutical Co. Ltd.
Suita-shi
Osaka 564-0032 (JP)**

(72) Inventors:
 • **SHIBATA, Takashi
  Suita-shi
  Osaka 564-0032 (JP)**
 • **ABE, Koji
  Suita-shi
  Osaka 564-0032 (JP)**

(74) Representative: **Kaiser, Magnus et al
Lemcke, Brommer & Partner
Patentanwälte
Bismarckstrasse 16
76133 Karlsruhe (DE)**

(54) **CHLORINE DIOXIDE GENERATING COMPOSITION**

(57)   A chlorine dioxide generating composition capable of generating chlorine dioxide through reaction between a chlorite and an acid. The composition contains a solid form chlorite, a solid form acid, and a solid form, moisture slow-release agent capable of slowly releasing moisture retained therein.

EP 1 985 582 A1

**Description**

**Technical Field**

The present invention relates to a chlorine dioxide generating composition capable of generating chlorine dioxide through reaction between a chlorite and an acid.

**Background Art**

Chlorine dioxide is a gaseous substance at a room temperature and has a strong oxidizing power and a disinfecting property, so that it is used in various applications such as deodorant, mildew-proofing agent, bleaching agent, etc.

That is, with supply of chlorine dioxide to a predetermined space, it becomes possible to deodorize or prevent generation of mildew in that space.

Chlorine dioxide gas can be supplied to such space as above by disposing a chlorine dioxide generating composition within the predetermined space or e.g. within a ventilation duct in communication with that space.

One conventional example of such chlorine dioxide generating composition comprises a gelled aqueous solution of chlorite (see e.g. Patent Documents 1-3). With this chlorine dioxide generating composition, the composition takes in carbon dioxide in the air to generate carbonic acid within the composition and this carbonic acid reacts with the chlorite to generate chlorine dioxide gas.

There is also known a construction wherein an aqueous solution containing chlorite and citric acid is prepared in the form of gel and chlorite and citric acid are caused to react with each other to generate chlorine dioxide gas (see e.g. Patent Document 4).

Patent Document 1: Japanese Patent Application "Kokai" No. 57-22102
Patent Document 2: Japanese Patent Application "Kokai" No. 61-40803
Patent Document 3: Japanese Patent Application "Kokai" No. 61-181532
Patent Document 4: Japanese Patent No. 3110724

**Disclosure of the Invention**

**Problem To Be Solved By Invention**

However, with the chlorine dioxide generating compositions described in Patent Documents 1-3, chlorine dioxide gas generated through the reaction between carbonic acid and chlorite is very low in its concentration, so they can be used only in a very small space such as a refrigerator.

On the other hand, with the chlorine dioxide generating composition described in Patent Document 4, through the reaction between chlorite and citric acid, chlorine dioxide gas can be generated in a relatively high concentration. However, as the chlorite and the citric acid are already dissolved in water, the reactivity is high so that it is very difficult to control the generation amount of carbon dioxide gas.

That is, with this chlorine dioxide generating composition, at an initial stage of its use, the reaction will proceed rapidly, so that a large amount of carbon dioxide can be generated. Yet, with lapse of a few days from the start of its use, the generation amount of carbon dioxide decreases. Therefore, it has been difficult to maintain the deodorizing effect or mildew generation preventing effect for an extended period of time.

The present invention has been made in view of the above-described state of the art and is intended to provide a chlorine dioxide generating composition which does not restrict its applicable space in particular and which can provide various effects of chlorine dioxide gas for an extended period of time.

**Means To Solve Problem**

For accomplishing the above-noted object, according to a first characterizing feature of a chlorine dioxide generating composition of the present invention, there is provided a chlorine dioxide generating composition capable of generating chlorine dioxide through reaction between a chlorite and an acid, wherein the composition contains a solid form chlorite, a solid form acid, and a solid form, moisture slow-release agent capable of slowly releasing moisture retained therein.

The chlorine dioxide generating composition of the above-described construction contains a solid form, moisture slow-release agent which is capable of slowly releasing moisture retained therein. In the context of this specification, the term "moisture" refers to steam (gas), water (liquid), ice (solid) or any mixture thereof containing these steam, water, ice at desired ratios.

With the chlorine dioxide generating composition of the above-described construction, the moisture slow-release agent slowly releases a small amount of moisture, which dissolves the solid form chlorite and the solid form acid, respectively, thereby generating a high concentration of chlorite solution in water and a high concentration of acid solution. And, as these highly concentrated chlorite water solution and acid solution reach with each other, a chlorine dioxide gas can be generated in a high concentration.

As described above, the chlorine dioxide generating composition of the above-described construction can supply chlorine dioxide gas in a high concentration. Therefore, the composition can be used not only in such a limited space as a refrigerator, but also in such a large space as an indoor space livable by humans. And, there is no particular limit to the size of the space applicable.

Further, as the chlorite water solution and acid solution in high concentrations can be generated little by little by with lapse of time by the moisture slowly released from the moisture slow-release agent., the condition of generating chlorine dioxide gas in the high concentration can be maintained for a long time.

Therefore, the chlorine dioxide generating composition of the above construction is free from the problem of the chlorine dioxide generating composition of the prior art, i.e. the completion of most of the reaction at the initial stage of its use, thus completing generation of most of the chlorine dioxide, and the inventive composition is capable of maintaining the various effects of chlorine dioxide gas, such as the deodorizing effect, the mildew generation preventive effect, etc. for an extended period of time.

According to a second characterizing feature of the present invention, the composition further contains a solid form deliquescent agent.

With this construction, due to the presence of the solid form deliquescent material contained therein, the composition can positively taken in moisture present in the ambient air. Then, this moisture taken in by the solid form deliquescent agent will migrate into the moisture slow-release agent to be temporarily retained therein and then can be released therefrom.

Therefore, the chlorine dioxide generating composition of this construction is capable of obtaining efficiently from the air the moisture needed for dissolution of the solid form chlorite and the solid form acid, so that the high concentration chlorine dioxide gas can be generated in stable manner.

According to a third characterizing feature of the present invention, the composition further contains liquid water and a gelling agent.

The liquid water contained in the chlorine dioxide generating composition of the above construction can be gelled by the gelling agent. Then, a portion of this gelled water will migrate into the moisture slow-release agent to be temporarily retained therein and then can be released therefrom.

That is to say, with the chlorine dioxide generating composition of the above-described construction, the composition can obtain, in an efficient manner, the moisture needed for the dissolution of the solid form chlorite and solid form acid, from the gelled liquid water. Then, the generation of chlorine dioxide gas can be less dependent upon the humidity of the ambient space. As result, the high concentration chlorine dioxide gas can be generated in a stable manner even in a dry space.

The dissolution of the solid form chlorite and solid form acid by the gelled water progresses much more slowly than dissolution thereof with non-gelled, common liquid water. Therefore, with the chlorine dioxide generating composition of the above-described construction, it is possible to restrict rapid reaction between chlorite and acid.

And, in case the composition contains the solid form deliquescent agent, the moisture needed for the dissolution of the solid form chlorite and solid form acid can be obtained not only from the gelled water, but also from the moisture present in the air. Therefore, even if the gelled water is lost due to e.g. evaporation, the solid form deliquescent agent can continue to take in moisture from the ambient air, so that the moisture needed for the dissolution of the solid form chlorite and solid form acid can be obtained in a stable and reliable manner. Consequently, the high concentration chlorine dioxide gas can be generated in a stable manner for an extended period of time.


**Best Mode of Embodying the Invention**

The chlorine dioxide generating composition of the present invention can be manufactured by mixing, with using a known mixing technique, a solid form chlorite, a solid form acid, and a solid form moisture slow-release agent capable of slowly releasing moisture retained therein, at an appropriate respective mixing ratios.

Further, if needed, in addition to the above-mentioned components, the composition can further contain at least one of water (liquid), a gelling agent, and a solid form deliquescent agent.


(Solid Form Chlorite)

Some non-limiting examples of the solid form chlorite usable in the chlorine dioxide generating composition of the present invention include an alkali metal salt of chlorite, such as sodium chlorite, potassium chlorite, etc., and an alkaline earth metal salt of chlorite, such as calcium chlorite, magnesium chlorite, etc.

The content of the solid form chlorite in the chlorine dioxide generating composition of the present invention ranges preferably from 5 weight % to 25 weight %. If the content of the solid form chlorite is below 5 weight %, generation of chlorine dioxide gas in a high concentration becomes difficult. On the other hand, if the content of the solid form chlorite exceeds 25 weight %, the chlorine dioxide generating composition of the invention will be classified as a dangerous substance, which is subject to a legal restriction concerning handling of dangerous substances. As a result, a special qualification will become needed for its handling, thus resulting in difficulty in handling.

(Solid Form Acid)

Some non-limiting examples of the solid form acid usable in the chlorine dioxide generating composition of the present invention include sodium dihydrogen phosphate, citric acid, tartaric acid, etc.

The content of the solid form acid in the chlorine dioxide generating composition of the present invention should be such that the acid, when used, reacts with the chlorite to a satisfactory degree to generate chlorine dioxide gas efficiently. More particularly, it is preferred that this content be an amount which can be theoretically derived from a chemical reaction formula, i.e. the minimal amount required for its reaction with the entire amount of chlorite.

If the content of the solid form acid is too low, a large amount of un-reacted chlorite will be left. On the other hand, if the content of the solid form acid is too high, a large amount of un-reacted acid will be left. In either case, there can occur inconvenience in disposing of the used chlorine dioxide generating composition.

For instance, if sodium chlorite ($NaClO_2$) is employed as the chlorite and citric acid (tricarboxylic acid) is employed as the solid form acid, in theoretical amounts, 15 mol (1358g) of sodium chlorite will react with 4 mol (768g) of citric acid, to generate 12 mol of chlorine dioxide (Formula 1).

(Formula 1) $\quad 15\ NaClO_2 + 4\ C_6H_8O_7 \rightarrow 12ClO_2 + 4C_6H_5Na_3O_7 + 3NaCl + 6H_2O$

(Moisture Slow-Release Agent)

The solid form, moisture slow-release agent usable in the chlorine dioxide generating composition of the invention is not particularly limited, but can be any solid substance having good moisture retaining power. This moisture slow-release agent retains moisture therein and releases a portion of the retained moisture through its evaporation. This moisture slow-release agent can be particles of natural mineral. Some preferred examples of natural mineral or ore are clay mineral, moisture-containing mineral, etc. Some specific examples are diatom earth, zeolite, kaolin, pearlite, bentonite, etc.

Preferably, the content of the moisture slow-release agent used in the chlorine dioxide generating composition of the present invention is such an amount capable of appropriately adsorbing an amount of moisture contained in this chlorine dioxide generating composition. If the content of the moisture slow-release agent is too low, there is the risk of liquid leak from the chlorine dioxide generating composition of the present invention. On the other hand, if the content of the moisture slow-release agent is too high, this can cause a difficulty in supplying the moisture for the dissolution of the solid form chlorite and the solid form acid, which difficulty leads to difficulty in the reaction between the chlorite and the acid, thus resulting in reduction in the concentration of the chlorine dioxide gas generated.

Incidentally, in the context of this specification, the term "moisture" refers to steam (gas), water (liquid), ice (solid) or any mixture thereof containing these steam, water, ice at desired ratios.

(Solid Form Deliquescent Agent)

Some non-limiting examples of the solid form deliquescent agent useable in the chlorine dioxide generating composition of the present invention include deliquescent inorganic compounds such as aluminum chloride, calcium chloride, magnesium chloride, potassium monohydrogen phosphate, and deliquescent organic compounds such as glycerin.

The content of the solid form deliquescent agent usable in the chlorine dioxide generating composition of the present invention can be appropriately adjusted in accordance with the purpose of the use, such as the size of the space, the use period, etc. If the content of the solid form deliquescent agent is increased, the response speed will increase, so that the concentration of the chlorine dioxide gas will be higher. However, the usable period of the chlorine dioxide generating composition of the invention will be shorter.

(Gelling Agent)

Some non-limiting examples of the gelling agent usable in the chlorine dioxide generating composition of the present invention are water-absorbent resins of the starch type, the cellulose type, and the synthetic polymer type.

Preferably, the content of the gelling agent used in the chlorine dioxide generating composition of the present invention should be made appropriate so as not to interfere with generation of chlorine dioxide for an extended period of time and also not to invite cost increase.

If the content of the gelling agent is too low, the gelatification will be insufficient, so that there can occur rapid and violent reaction between chlorite and the acid. As a result, it becomes difficult to maintain the generation of chlorine dioxide for an extended period of time. On the other hand, if the content of the gelling agent is too high, this will result in increase in the manufacture costs, hence, economical disadvantage.

(Applicable Space)

The chlorine dioxide generating composition of the present invention can be used not only in such a limited space as a refrigerator, but also in such a large space as an indoor space livable by humans. And, there is no particular limit to the size of the space applicable.

The chlorine dioxide generating composition of the present invention can be put in e.g. a bag made of an air-permeable non-woven fabric and this can be set in a space with ventilation such as an exit of an air conditioner, an air conditioner duct, etc. so as to supply chlorine dioxide gas to the predetermined space. Further, this chlorine dioxide generating composition can be put in a container having an opening and this can be set in a predetermined space of limited ventilation, so as to supply chlorine dioxide gas to the predetermined space.

Examples

Next, the present invention will be described more specifically with reference to some examples. It is understood, however, that the present invention is not limited thereto.

(1) Determination of variation over time of the concentration of chlorine dioxide gas generated by the chlorine dioxide generating gas of the present invention.

Respective particles not containing free moisture, of 80% sodium chlorite (solid form chlorite), sodium dihydrogen phosphate or citric acid (solid form acid), porous particulate powder WATERREFINE N (moisture slow-release agent: available from Kyoei Calcium Co., Ltd.), calcium chloride (solid form deliquescent agent), gelling agent, AQUATIC (gelling agent available from Nippon Shokubai Co., Ltd.) were used in the mixing ratios shown in Table 1 below and mixed in a dry room to make samples of the chlorine dioxide generating composition (Examples 1-8).

Each sample made as above was charged in a container having two openings. And, ambient air was introduced through one opening into the container at the ratio of 5L/min. and variation over time of the concentration (ppm) of generated chlorine dioxide gas from the other opening was determined by a detector tube.

Incidentally, as comparison examples, like the conventional chlorine dioxide generating composition described in Patent Document 4, there were made samples comprised of gelled aqueous solutions containing sodium chlorite and sodium dihydrogen phosphate (Comparison Examples 1-2). And, like the chlorine dioxide generating composition of the present invention, variation over time of the concentration (ppm) of generated chlorine dioxide gas from the other opening was determined by a detector tube.

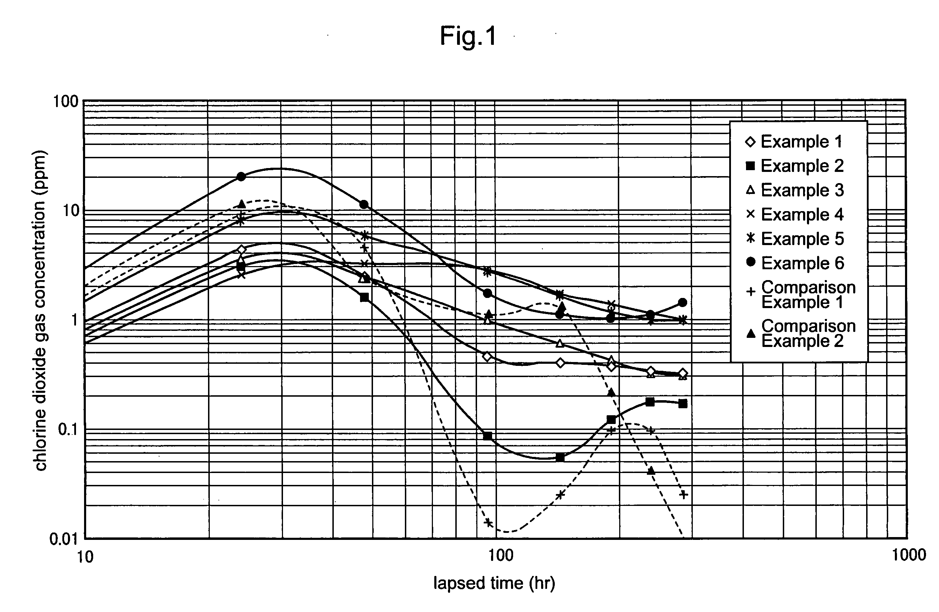

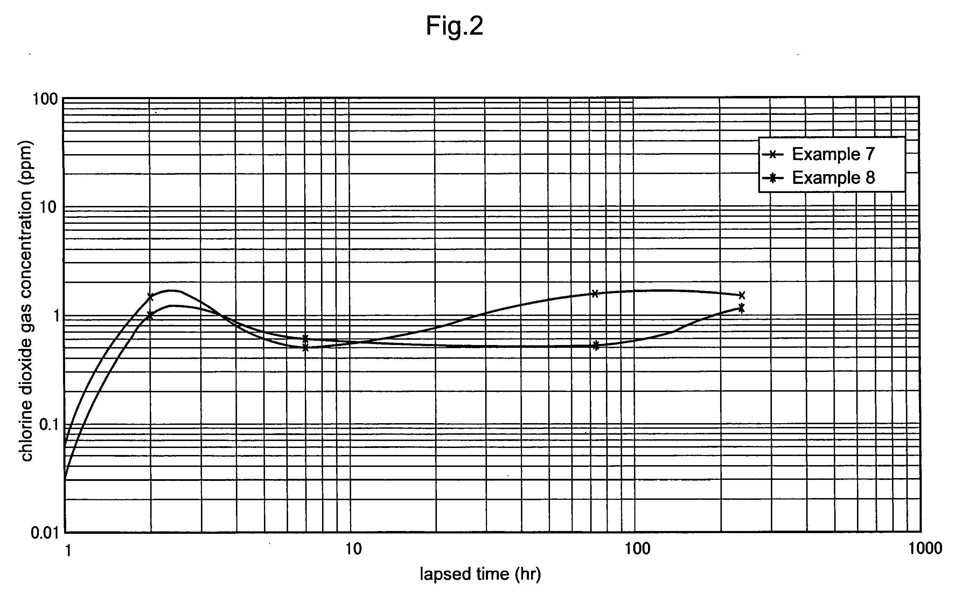

The results are shown in Fig. 1 (Examples 1-6, Comparison Examples 1-2) and Fig. 2 (Examples 7-8).

[Table 1]

| Sample | 80% sodium chlorite (g) | sodium dihydrogen phosphate | citric acid (g) | porous particulate powder (g) | calcium chloride (g) | gelling agent AQUALIC (g) | water (g) |
|---|---|---|---|---|---|---|---|
| Example 1 | 4.26 | 2.04 | 0.0 | 23.7 | 0.0 | 0.0 | 0.0 |
| Example 2 | 4.26 | 2.04 | 0.0 | 23.7 | 0.6 | 0.0 | 0.0 |
| Example 3 | 4.26 | 2.04 | 0.0 | 20.7 | 3.0 | 0.0 | 0.0 |
| Example 4 | 4.26 | 2.04 | 0.0 | 17.7 | 6.0 | 0.0 | 0.0 |
| Example 5 | 4.26 | 2.04 | 0.0 | 17.7 | 0.0 | 0.6 | 5.4 |

(continued)

| Sample | 80% sodium chlorite (g) | sodium dihydrogen phosphate | citric acid (g) | porous particulate powder (g) | calcium chloride (g) | gelling agent AQUALIC (g) | water (g) |
|---|---|---|---|---|---|---|---|
| Example 6 | 4.26 | 2.04 | 0.0 | 11.7 | 10.0 | 0.6 | 5.4 |
| Example 7 | 12.0 | 0.0 | 5.5 | 31.0 | 11.0 | 0.0 | 0.0 |
| Example 8 | 12.0 | 6.0 | 0.0 | 31.0 | 11.0 | 0.0 | 0.0 |
| Comparison Example 1 | 4.26 | 2.04 | 0.0 | 0.0 | 0.0 | 2.8 | 21.6 |
| Comparison Example 2 | 8.40 | 3.00 | 0.0 | 0.0 | 0.0 | 4.05 | 51.2 |

In Table 2 below, there are shown maximum concentrations (A), and concentrations (B) after 300 hours of chlorine dioxide gas, and ratios therebetween (=A/B) for the respective samples.

[Table 2]

| Sample | A: maximum concentration (ppm) | B: concentration after 300 hrs (ppm) | A/B |
|---|---|---|---|
| | | | |
| Example 1 | 5.0 | 0.32 | 15.6 |
| Example 2 | 3.3 | 0.17 | 19.4 |
| Example 3 | 4.0 | 0.30 | 13.3 |
| Example 4 | 3.3 | 0.95 | 3.5 |
| Example 5 | 9.7 | 0.98 | 9.9 |
| Example 6 | 24.0 | 1.40 | 17.1 |
| Example 7 | 1.8 | 1.50 | 1.2 |
| Example 8 | 1.1 | 1.05 | 1.0 |
| Comparison Example 1 | 10.0 | 0.03 | 333 |
| Comparison Example 2 | 11.0 | 0.01 | 1100 |

Example 1 does not contain calcium chloride (solid form deliquescent agent). Examples 2-4 contain calcium chloride, with the contents of the calcium chloride in the respective samples are: 1.96 weight % (Example 2), 10 weight % (Example 3) and 20 weight % (Example 4).

As shown in Example 3 in Fig. 1 and the graph of Example 4, it may be seen that chlorine dioxide gas can be generated in a more stable manner for an extended period of time especially in the case of the calcium chloride content from 10 weight % to 20 weight %.

Example 5 does not contain calcium chloride, but contains water and gelling agent. Example 6 contains calcium chloride, water and gelling agent. As shown in Fig. 1, in Example 5 and Example 6, as compared with the above-described case of Examples 1-4, the concentrations of the chlorine dioxide gas generated at the initial stage were found higher. It is believed that this is because the moisture contained in the calcium chloride or the gelling agent affects the sodium chlorite and the sodium dihydrogen phosphate.

Examples 7 and 8 contain calcium chloride in 20 weight %. Example 7 contains citric acid and Example 8 contains sodium dihydrogen phosphate. As shown in Fig. 2, it was seen that chlorine dioxide gas was generated in a stable manner for an extended period of time.

On the other hand, Comparison Examples 1-2 have constructions similar to the conventional chlorine dioxide generating composition described in Patent Document 4, hence not containing the moisture slow-release agent. As shown in Fig. 1, in Comparison Example 1 and Comparison Example 2, a great amount of chlorine dioxide gas was generated at the initial stage and the chlorine dioxide gas concentrations after 300 hours were significantly lowered.

(2) Deodorization Test

50g of sample of chlorine dioxide generating composition of Example 7 was put in a bag made of an air-permeable non-woven PET/PP fabric sized 10 cm x 12 cm and sealed by heat sealing. This bag charged with this chlorine dioxide generating composition was set inside an indoor unit of an air conditioner (FHYCP63H: DAIKIN INDUSTRIES, Ltd.). Then, the air conditioner was operated, so that the air in the indoor space ( 5 m x 8 m x 2.8 m =12 $m^3$) was circulated on the condition of 15 $m^3$/min. for about 8 hours a day, for the total time of 500 hours approximately. The air conditioner was operated only during the daytime working hours, and was stopped at night.

Incidentally, the total use time of the chlorine dioxide generating composition sample of Example 7 amounts to about 1500 hours (about 2 months).

The air conditioner starting odor, cigarette smoking odor, chlorine dioxide odor, chlorine dioxide gas concentration in the indoor space were respectively determined after lapse of a predetermined time period. As for the air conditioner starting odor, cigarette smoking odor, chlorine dioxide odor, sensory evaluations were conducted by several panelists to evaluate the strength of the respective odor. Each evaluation was made in the six-step evaluation from 0 to 5, with 0 being no odor, the greater the number, the stronger the odor.

Further, as to the chlorine dioxide concentration (ppb), the determination was made with using an electrolytic current type chlorine dioxide gas densitometer ("AM-ClO$_2$ type" DAISO ENGINEERING CO., LTD.) with some modification thereof.

As to the chlorine dioxide generation amount (mg/hr), based on the chlorine dioxide gas concentration determined with using the above chlorine dioxide gas densitometer, the amount was calculated, with setting the indoor air ventilation amount: 112 $m^3$/hr and the room temperature: 20°C. For instance, if the chlorine dioxide concentration is determined as 20 ppb, then, the chlorine dioxide generation amount (mg/hr) is:

$$20 \text{ (ppb)} \times 10^{-6} \times 67.5 \times 1000 / (0.082 \times 293) \times 112 \text{ (m}^3\text{/hr)} = 6.3 \text{ (mg/hr)}$$

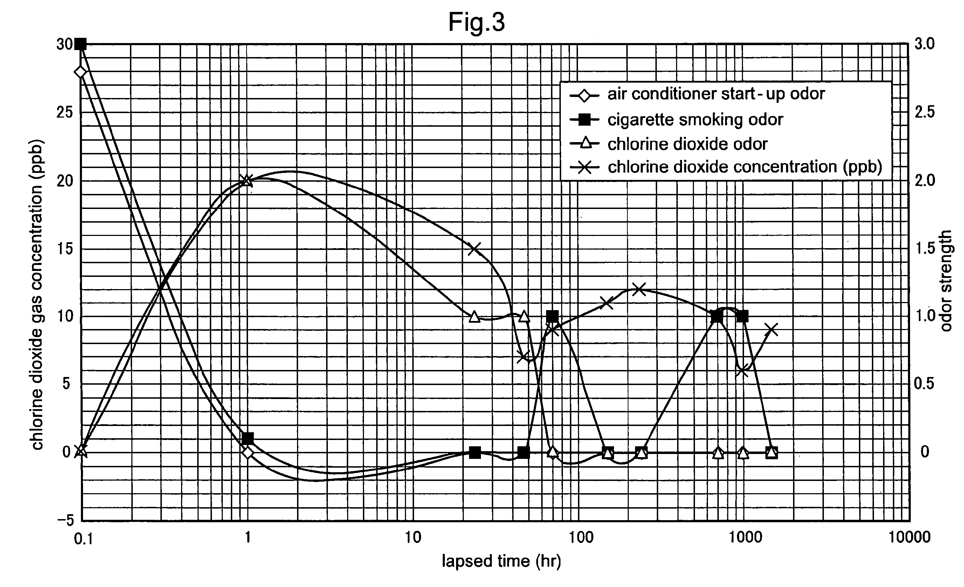

(incidentally, the value 67.5 represents the molecular weight of chlorine dioxide, the value 0.082 represents gas constant; and the value 293 represents the absolute temperature, respectively). The results are shown in Table 3 and Fig. 3 below.

[Table 3]

| lapsed time (hr) | air conditioner start-up odor | cigarette smoking odor | chlorine dioxide odor | chlorine dioxide concentration (ppb) | chlorine dioxide generation amount (mg/hr) |
|---|---|---|---|---|---|
| 0 | 3 | 3 | 0 | 0 | 0.0 |
| 1 | 0 | 1 | 2 | 20 | 6.3 |
| 24 | 0 | 0 | 1 | 15 | 4.7 |
| 48 | 0 | 0 | 1 | 7 | 2.2 |
| 72 | 0 | 1 | 1 | 9 | 2.8 |
| 150 | 0 | 0 | 0 | 11 | 3.5 |
| 240 | 0 | 1 | 0 | 12 | 3.8 |
| 696 | 0 | 1 | 0 | 10 | 3.1 |
| 1000 | 0 | 1 | 0 | 6 | 1.9 |
| 1500 | 0 | 1 | 0 | 9 | 2.8 |

The amount of chlorite contained in 50 g of Sample of Example 7 is:

$$12 \times 50 / 59.5 \times 0.8 = 8.0 \text{ g } (0.088 \text{ mol})$$

and

The amount of citric acid contained therein is:

$$5.5 \times 50 / 59.5 = 4.6 \text{ g } (0.023 \text{ mol}).$$

Therefore, theoretically, in the case of sample of Example 7, the amount of chlorine dioxide which can be generated when the entire amount of citric acid reacts with the sodium chlorite is:

$$0.023 \times 3 \times 67.5 = 4.6 \text{ g}$$

Here, it is believed that with the chlorine dioxide generating composition of Example 7, the composition can generate chlorine dioxide more easily if the air can be positively introduced therein. Therefore, let us assume that chlorine dioxide is generated only during the operating hours (8 hours during daytime) of the air conditioner. And, for the sake of convenience, there were calculated the average value of the chlorine dioxide generation amounts (mg/hr) shown in Table 3 (3.1 = (0.0 + 6.3 + 4.7 + 2.2 + 2.8 + 3.5 + 3.8 + 3.1 + 1.9 + 2.8) /10) and from this average value (3.1 mg/hr), the total generation amount of chlorine dioxide in the operating hours (500 hours) of the air conditioner was calculated as: 1.5 g (= 3.1 x 500 / 1000).

Therefore, the amount of chlorine dioxide generated in the period of about 2 months when the chlorine dioxide generating composition of Example 7 is used in the above-described method is about 32. 6 (= 1.5 /4.6 x 100)% of the theoretically generated amount of chlorine dioxide. This shows that the composition still retains high chlorine dioxide generating ability. Therefore, if this condition is maintained, it is estimated that the sample of Example 7 can be used for as long as about 6 months.

As described above, it has been confirmed that the chlorine dioxide generating composition of the present invention is capable of generating chlorine dioxide gas in a high concentration for an extended period of time, and as a result, the various effects (deodorization effect) of the chlorine dioxide gas can be maintained for an extended period of time.

**Industrial Applicability**

The chlorine dioxide generating composition of the present invention is capable of generating chlorine dioxide having strong oxidizing power and disinfecting property, so this composition can be used in a various of applications, such as deodorant, mildew-proofing agent, bleaching agent, etc.

**Brief Description of the Drawings**

[Fig. 1] a diagram showing change over time of the concentration of chlorine dioxide gas generated from the chlorine dioxide generating composition according to the present invention,

[Fig. 2] a diagram showing change over time of the concentration of chlorine dioxide gas generated from the chlorine dioxide generating composition according to the present invention, and

[Fig. 3] a diagram showing the concentration of chlorine dioxide gas generated from the chlorine dioxide generating composition according to the present invention and its deodorizing effect.

**Claims**

**1.** A chlorine dioxide generating composition capable of generating chlorine dioxide through reaction between a chlorite and an acid,

wherein the composition contains a solid form chlorite, a solid form acid, and a solid form, moisture slow-release agent capable of slowly releasing moisture retained therein.

2. The chlorine dioxide generating composition according to claim 1, wherein the composition further contains a solid form deliquescent agent.

3. The chlorine dioxide generating composition according to claim 1 or 2, wherein the composition further contains liquid water and a gelling agent.

4. The chlorine dioxide generating composition according to claim 1 or 2, wherein said solid form chlorite is selected from the group consisting of sodium chlorite, potassium chlorite, calcium chlorite, and magnesium chlorite.

5. The chlorine dioxide generating composition according to claim 1 or 2, wherein the composition contains said solid form chlorite in from 5 to 25 weight %.

6. The chlorine dioxide generating composition according to claim 1 or 2, wherein said solid acid is selected from the group consisting of sodium dihydrogen phosphate, citric acid, and tartaric acid.

7. The chlorine dioxide generating composition according to claim 1 or 2, wherein said solid form, moisture slow-release agent is selected from the group consisting of diatom earth, zeolite, kaolin, pearlite, and bentonite.

8. The chlorine dioxide generating composition according to claim 2, wherein said solid form deliquescent agent is selected from the group consisting of aluminum chloride, calcium chloride, magnesium chloride, potassium monohydrogen phosphate, and glycerin.

9. The chlorine dioxide generating composition according to claim 8, wherein the composition contains said calcium chloride in from 10 to 20 weight %.

10. The chlorine dioxide generating composition according to claim 3, wherein said gelling agent is selected from the group consisting of water-absorbent resins of the starch type, the cellulose type, and the synthetic polymer type.

Fig.1

Legend:
- ◇ Example 1
- ■ Example 2
- △ Example 3
- × Example 4
- ✳ Example 5
- ● Example 6
- + Comparison Example 1
- ▲ Comparison Example 2

Y-axis: chlorine dioxide gas concentration (ppm)

X-axis: lapsed time (hr)

## Fig.2

## Fig.3

chlorine dioxide gas concentration (ppb)

odor strength

lapsed time (hr)

- ◇ air conditioner start-up odor
- ■ cigarette smoking odor
- △ chlorine dioxide odor
- ✕ chlorine dioxide concentration (ppb)

EP 1 985 582 A1

**INTERNATIONAL SEARCH REPORT**

| International application No. |
| --- |
| PCT/JP2007/052580 |

A. CLASSIFICATION OF SUBJECT MATTER
*C01B11/02*(2006.01)i, *A01N25/12*(2006.01)i, *A01N59/00*(2006.01)i, *A01N59/08* (2006.01)i, *A01P3/00*(2006.01)i, *A61L9/01*(2006.01)i, *B01J7/00*(2006.01)i

According to International Patent Classification (IPC) or to both national classification and IPC

B. FIELDS SEARCHED

Minimum documentation searched (classification system followed by classification symbols)
C01B11/02, A01N25/12, A01N59/00, A01N59/08, A01P3/00, A61L9/01, B01J7/00

Documentation searched other than minimum documentation to the extent that such documents are included in the fields searched
Jitsuyo Shinan Koho     1922-1996   Jitsuyo Shinan Toroku Koho   1996-2007
Kokai Jitsuyo Shinan Koho   1971-2007   Toroku Jitsuyo Shinan Koho   1994-2007

Electronic data base consulted during the international search (name of data base and, where practicable, search terms used)
JST7580(JDream2), JSTPlus(JDream2)

C. DOCUMENTS CONSIDERED TO BE RELEVANT

| Category* | Citation of document, with indication, where appropriate, of the relevant passages | Relevant to claim No. |
| --- | --- | --- |
| X<br>Y<br>A | JP 63-246304 A  (Kabushiki Kaisha Health Kosan),<br>13 October, 1988 (13.10.88),<br>Claims 1, 2; examples 1 to 3<br>(Family: none) | 1,4,5,7<br>2,8,9<br>3,6,10 |
| X<br>Y<br>A | JP 2001-501086 A  (Bernard Technologies, Inc.),<br>30 January, 2001 (30.01.01),<br>Example 3, 4<br>& US 5965264 A       & WO 1998/011776 A1 | 1,4,7<br>2,8,9<br>3,5,6,10 |
| Y | JP 2001-516213 A  (Engelhard Corp.),<br>25 September, 2001 (25.09.01),<br>Claims 20 to 37; examples 6, 7, 10<br>& WO 1998/038865 A1 | 2,8,9 |

| ☒ | Further documents are listed in the continuation of Box C. | ☐ | See patent family annex. |
| --- | --- | --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>18 April, 2007 (18.04.07) | Date of mailing of the international search report<br>01 May, 2007 (01.05.07) |
| --- | --- |
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

International application No.

PCT/JP2007/052580

C (Continuation).    DOCUMENTS CONSIDERED TO BE RELEVANT

| Category* | Citation of document, with indication, where appropriate, of the relevant passages | Relevant to claim No. |
|---|---|---|
| A | JP 64-071804 A  (Shikoku Kasei Co., Ltd.), 16 March, 1989 (16.03.89), Full descriptions (Family: none) | 1-10 |
| A | JP 60-206425 A  (Kabushiki Kaisha Enkura Bijinesu), 18 October, 1985 (18.10.85), Full descriptions (Family: none) | 1-10 |
| A | JP 57-022102 A  (Daimaru Kogyo, Ltd. et al.), 05 February, 1982 (05.02.82), Full descriptions (Family: none) | 1-10 |
| A | JP 04-349104 A  (Takasugi Seiyaku Kabushiki Kaisha), 03 December, 1992 (03.12.92), Full descriptions (Family: none) | 1-10 |

Form PCT/ISA/210 (continuation of second sheet) (April 2005)

**REFERENCES CITED IN THE DESCRIPTION**

**Patent documents cited in the description**

- JP 57022102 A
- JP 61040803 A
- JP 61181532 A
- JP 3110724 B